# EUROPEAN PATENT SPECIFICATION

(11) **EP 2 244 953 B1**
(45) Date of publication and mention of the grant of the patent: **05.10.2011**
(21) Application number: 09701373.4
(22) Date of filing: 06.01.2009
(51) Int. Cl.: B65D 25/00, C11D 3/00, G02B 1/04, A61L 12/08

(54) **PACKAGING SOLUTIONS**
VERPACKUNGSLÖSUNGEN
SOLUTIONS DE CONSERVATION

(30) Priority: 09.01.2008 US 19864 P
(43) Date of publication of application: 03.11.2010
(73) Proprietor: Bausch & Lomb Incorporated, Rochester, NY 14604-2701 (US)
(72) Inventor: LAI, Yu-Chin, Pittsford NY 14534 (US); LANG, Weihong, Amston CT 06231 (US)
(74) Representative: Glas, Holger
(86) International application number: PCT/US2009/030198
(87) International publication number: WO 2009/089206

(56) References cited:
- WO-A-98/30248
- WO-A-98/55155
- WO-A-2006/038080
- WO-A-2006/053164

## Description

The present invention generally relates to packaging solutions for ophthalmic devices such as contact lenses.

### 2. Description of Related Art

Blister-packs and glass vials are typically used to individually package each soft contact lens for sale to a customer. Saline or deionized water is commonly used to store the lens in the blister-packs, as mentioned in various patents related to the packaging or manufacturing of contact lenses. Because lens material may tend to stick to itself and to the lens package, packaging solutions for blister-packs have sometimes been formulated to reduce or eliminate lens folding and sticking. For this reason, polyvinyl alcohol (PVA) has been used in contact-lens packaging solutions.

It has been stated that if a lens is thoroughly cleaned before insertion, lacrimal fluid can adequately wet the lens. Furthermore, the difficulties of adding a surfactant to a packaging solution, including the possibility of lowering shelf-life and/or adverse reactions during heat sterilization, have further limited the use of surfactants in a packaging solution for the purpose of providing any possible or marginal effect on lens comfort. It is only after a lens has been worn, when proteins or other deposits have formed on the surface of the lens, that surfactants have been used in standard lens-care solutions.

It is highly desirable that contact lens be as comfortable as possible for wearers. Manufacturers of contact lenses are continually working to improve the comfort of the lenses. Nevertheless, many people who wear contact lenses still experience dryness or eye irritation throughout the day and particularly towards the end of the day. An insufficiently wetted lens at any point in time will cause significant discomfort to the lens wearer. Although wetting drops can be used as needed to alleviate such discomfort, it would certainly be desirable if such discomfort did not arise in the first place.

Poloxamine and poloxamers are examples of non-ionic surfactants having one or more poly(oxyalkylene) chains. Poloxamines and poloxamers are well-known wetting and lubricating agents for contact lenses and have been used in lens wetting drops and in lens-care solutions for treating lenses after use or while in use in the eye. For example, U.S. Patent No. 4,786,436 disclose poloxamine as a wetting agent. Contact-lens rewetting drops containing surfactants such as poloxamine and poloxamer have been used to make contact lens wear more comfortable, to soothe the eyes, and to moisten lenses to minimize dryness. Surfactants such as poloxamine, poloxamer, and tyloxapol have been used in multi-purpose solutions, for cleaning, wetting, and storing lenses.

Certain combinations of poly(oxyalkylene) surfactants have also been disclosed for use in the eye to preventively clean lenses and inhibit deposits. For example, U.S. Patent No. 5,209,865 discloses the combination of certain poloxamers and poloxamines to maintain clean lenses in the eye.

U.S. Patent No. 6,440,366 ("the '366 patent") discloses a package containing a contact lens suitable for immediate use which comprises (a) a solution comprising a non-ionic surfactant that is a compound comprising at least 90 weight percent of poly(oxyethylene) and poly(oxypropylene) segments, in one or more block copolymer chains, and (b) an effective amount of a tonicity adjusting agent such that the solution has an osmolality of 200 to 400 mOsm/kg; wherein the solution has a pH of about 6 to 8 and is heat sterilized and lacks an effective disinfecting amount of a disinfecting agent. The '366 patent further discloses that the surfactant is a poly(oxypropylene)-poly(oxyethylene) adduct of ethylene diamine.

It would be desirable to provide an improved packaging system for ophthalmic devices such as a contact lens such that the lens would be comfortable to wear in actual use and allow for extended wear of the lens without irritation or other adverse effects to the cornea.

### SUMMARY OF THE INVENTION

In accordance with one embodiment of the present invention, a method of preparing a package comprising a storable, sterile ophthalmic device as defined in claim 1 is provided comprising:
(a) immersing an ophthalmic device in an aqueous packaging solution comprising a hydrophilic polymer having one or more non-ethylenically-unsaturated carboxylic acid terminated groups, wherein the solution has an osmolality of at least about 200 mOsm/kg and a pH in the range of about 6 to about 9;
(b) packaging the solution and the ophthalmic device in a manner preventing contamination of the device by microorganisms; and
(c) sterilizing the packaged solution and ophthalmic device.

In accordance with a second embodiment of the present invention, a packaging system for the storage of an ophthalmic device as defined in claim 11 is provided comprising a sealed container containing one or more unused ophthalmic devices immersed in an aqueous packaging solution comprising a hydrophilic polymer having one or more non-ethylenically-unsaturated carboxylic acid terminated groups, wherein the solution has an osmolality of at least about 200 mOsm/kg, a pH of about 6 to about 9 and is heat sterilized.

The aqueous packaging solutions of the present invention containing at least a hydrophilic polymer having one or more non-ethylenically-unsaturated carboxylic acid terminated groups is believed to provide a more uniform coating on the surface of an ophthalmic device such as a contact lens thereby resulting in improved lubricity of the lens. Thus, the lens will be more comfortable to wear in actual use and would allow for the extended wear of the lens without irritation or other adverse effects to the cornea.

### DETAILED DESCRIPTION OF THE PREFERRED EMBODIMENTS

The present invention provides a packaging system for the storage of ophthalmic devices intended for direct contact with body tissue or body fluid. As used herein, the term "ophthalmic device" refers to devices that reside in or on the eye. These lenses can provide optical correction, wound care, drug delivery, diagnostic functionality or cosmetic enhancement or effect or a combination of these properties. Representative examples of such devices include, but are not limited to, soft contact lenses, e.g., a soft, hydrogel lens; soft, non-hydrogel lens and the like, hard contact lenses, e.g., a hard, gas permeable lens material and the like, intraocular lenses, overlay lenses, ocular inserts, optical inserts and the like. As is understood by one skilled in the art, a lens is considered to be "soft" if it can be folded back upon itself without breaking.

The ophthalmic devices can be any material known in the art capable of forming an ophthalmic device as described above. In one embodiment, an ophthalmic device includes devices which are formed from material not hydrophilic per se. Such devices are formed from materials known in the art and include, by way of example, polysiloxanes, perfluoropolyethers, fluorinated poly(meth)acrylates or equivalent fluorinated polymers derived, e.g., from other polymerizable carboxylic acids, polyalkyl (meth)acrylates or equivalent alkylester polymers derived from other polymerizable carboxylic acids, or fluorinated polyolefins, such as fluorinated ethylene propylene polymers, or tetrafluoroethylene, preferably in combination with a dioxol, e.g., perfluoro-2,2-dimethyl-1,3-dioxol. Representative examples of suitable bulk materials include, but are not limited to, Lotrafilcon A, Neofocon, Pasifocon, Telefocon, Silafocon, Fluorsilfocon, Paflufocon, Silafocon, Elastofilcon, Fluorofocon or Teflon AF materials, such as Teflon AF 1600 or Teflon AF 2400 which are copolymers of about 63 to about 73 mol % of perfluoro-2,2-dimethyl-1,3-dioxol and about 37 to about 27 mol % of tetrafluoroethylene, or of about 80 to about 90 mol % of perfluoro-2,2-dimethyl-1,3-dioxol and about 20 to about 10 mol % of tetrafluoroethylene.

In another embodiment, an ophthalmic device includes devices which are formed from material hydrophilic per se, since reactive groups, e.g., carboxy, carbamoyl, sulfate, sulfonate, phosphate, amine, ammonium or hydroxy groups, are inherently present in the material and therefore also at the surface of an ophthalmic device manufactured therefrom. Such devices are formed from materials known in the art and include, by way of example, polyhydroxyethyl acrylate, polyhydroxyethyl methacrylate (HEMA), polyvinyl pyrrolidone (PVP), polyacrylic acid, polymethacrylic acid, polyacrylamide, polydimethylacrylamide (DMA), polyvinyl alcohol and the like and copolymers thereof, e.g., from two or more monomers selected from hydroxyethyl acrylate, hydroxyethyl methacrylate, N-vinyl pyrrolidone, acrylic acid, methacrylic acid, acrylamide, dimethyl acrylamide, vinyl alcohol and the like. Representative examples of suitable bulk materials include, but are not limited to, Polymacon, Tefilcon, Methafilcon, Deltafilcon, Bufilcon, Phemfilcon, Ocufilcon, Focofilcon, Etafilcon, Hefilcon, Vifilcon, Tetrafilcon, Perfilcon, Droxifilcon, Dimefilcon, Isofilcon, Mafilcon, Nelfilcon, Atlafilcon and the like. Examples of other suitable bulk materials include balafilcon A, hilafilcon A, alphafilcon A, bilafilcon B and the like.

In another embodiment, ophthalmic devices include devices which are formed from material which are amphiphilic segmented copolymers containing at least one hydrophobic segment and at least one hydrophilic segment which are linked through a bond or a bridge member.

It is particularly useful to employ biocompatible materials herein including both soft and rigid materials commonly used for ophthalmic lenses, including contact lenses. In general, non-hydrogel materials are hydrophobic polymeric materials that do not contain water in their equilibrium state. Typical non-hydrogel materials comprise silicone acrylics, such as those formed bulky silicone monomer (e.g., tris(trimethylsiloxy)silylpropyl methacrylate, commonly known as "TRIS" monomer), methacrylate end-capped poly(dimethylsiloxane) prepolymer, or silicones having fluoroalkyl side groups (polysiloxanes are also commonly known as silicone polymers).

Hydrogels in general are a well-known class of materials that comprise hydrated, crosslinked polymeric systems containing water in an equilibrium state. Accordingly, hydrogels are copolymers prepared from hydrophilic monomers. In the case of silicone hydrogels, the hydrogel copolymers are generally prepared by polymerizing a mixture containing at least one device-forming silicone-containing monomer and at least one device-forming hydrophilic monomer. Either the silicone-containing monomer or the hydrophilic monomer can function as a crosslinking agent (a crosslinker being defined as a monomer having multiple polymerizable functionalities) or a separate crosslinker may be employed. Silicone hydrogels typically have a water content between about 10 to about 80 weight percent.

Representative examples of useful hydrophilic monomers include, but are not limited to, amides such as N,N-dimethylacrylamide and N,N-dimethylmethacrylamide; cyclic lactams such as N-vinyl-2-pyrrolidone; and (meth)acrylated poly(alkene glycols), such as poly(diethylene glycols) of varying chain length containing monomethacrylate or dimethacrylate end caps. Still further examples are the hydrophilic vinyl carbonate or vinyl carbamate monomers disclosed in U.S. Patent No. 5,070,215, and the hydrophilic oxazolone monomers disclosed in U.S. Patent No. 4,910,27. Other suitable hydrophilic monomers will be apparent to one skilled in the art. For example, 2-hydroxyethylmethacrylate (HEMA) is a well-known hydrophilic monomer that may be used in admixture with the aforementioned hydrophilic monomers.

The monomer mixtures may also include a second device-forming monomer including a copolymerizable group and a reactive functional group. The copolyermizable group is preferably an ethylenically unsaturated group, such that this device-forming monomer copolymerizes with the hydrophilic device-forming monomer and any other device-forming monomers in the initial device-forming monomer mixture. Additionally, the second monomer can include a reactive functional group that reacts with a complementary reactive group of the hydrophilic polymer having one or more non-ethylenically-unsaturated carboxylic acid terminated groups. In other words, after the device is formed by copolymerizing the device-forming monomer mixture, the reactive functional groups provided by the second device-forming monomers remain to react with a complementary reactive moiety of the hydrophilic polymer having one or more non-ethylenically-unsaturated carboxylic acid terminated groups.

Preferred reactive groups of the second device-forming monomers include epoxide groups. Accordingly, preferred second device-forming monomers are those that include both an ethylenically unsaturated group (that permits the monomer to copolymerize with the hydrophilic device-forming monomer) and the epoxide group (that does not react with the hydrophilic device-forming monomer but remains to react with the hydrophilic polymer having one or more non-ethylenically-unsaturated carboxylic acid terminated groups). Examples include glycidyl methacrylate, glycidyl acrylate, glycidyl vinylcarbonate, glycidyl vinylcarbamate, 4-vinyl-1-cyclohexene-1,2-epoxide and the like.

As mentioned, one preferred class of ophthalmic device substrate materials are silicone hydrogels. In this case, the initial device-forming monomer mixture further comprises a silicone-containing monomer. Applicable silicone-containing monomeric materials for use in the formation of silicone hydrogels are well known in the art and numerous examples are provided in U.S. Pat. Nos. 4,136,250; 4,153,641; 4,740,533; 5,034,461; 5,070,215; 5,260,000; 5,310,779; and 5,358,995. Specific examples of suitable materials for use herein include those disclosed in U.S. Patent Nos. 5,310,779; 5,387,662; 5,449,729; 5,512,205; 5,610,252; 5,616,757; 5,708,094; 5,710,302; 5,714,557 and 5,908,906.

Representative examples of applicable silicon-containing monomers include bulky polysiloxanylalkyl(meth)acrylic monomers. The term "monomer" and like terms as used herein denote relatively low molecular weight compounds that are polymerizable by, for example, free radical polymerization, as well as higher molecular weight compounds also referred to as "prepolymers", "macromonomers", and related terms. The term "(meth)" as used herein denotes an optional methyl substituent. Accordingly, terms such as "(meth)acrylate" denotes either methacrylate or acrylate, and "(meth)acrylic acid" denotes either methacrylic acid or acrylic acid.

An example of a bulky polysiloxanylalkyl(meth)acrylic monomer is represented by the structure of Formula I: 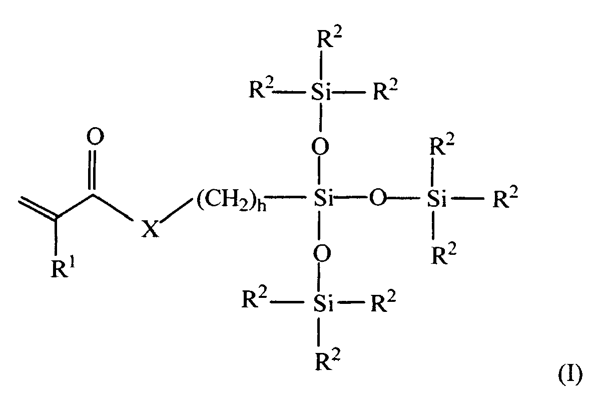 wherein X denotes -O- or -NR-; each R¹ independently denotes hydrogen or methyl; each R² independently denotes a lower alkyl radical, phenyl radical or a group represented by 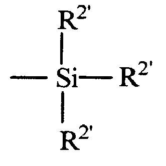 wherein each R² independently denotes a lower alkyl or phenyl radical; and h is 1 to 10.

Examples of bulky monomers are methacryloxypropyl tris(trimethyl-siloxy)silane or tris(trimethylsiloxy)silylpropyl methacrylate, sometimes referred to as TRIS and tris(trimethylsiloxy)silylpropyl vinyl carbamate, sometimes referred to as TRIS-VC and the like.

Such bulky monomers may be copolymerized with a silicone macromonomer, which is a poly(organosiloxane) capped with an unsaturated group at two or more ends of the molecule. U.S. Patent No. 4,153,641 discloses, for example, various unsaturated groups such as acryloxy or methacryloxy groups.

Another class of representative silicone-containing monomers includes, but is not limited to, silicone-containing vinyl carbonate or vinyl carbamate monomers such as, for example, 1,3-bis[4-vinyloxycarbonyloxy)but-1-yl]tetramethyl-disiloxane; 3-(trimethylsilyl)propyl vinyl carbonate; 3-(vinyloxycarbonylthio)propyl-[tris(trimethylsiloxy)silane]; 3-[tris(trimethylsiloxy)silyl]propyl vinyl carbamate; 3-[tris(trimethylsiloxy)silyl]propyl allyl carbamate; 3-[tris(trimethylsiloxy)silyl]propyl vinyl carbonate; t-butyldimethylsiloxyethyl vinyl carbonate; trimethylsilylethyl vinyl carbonate; trimethylsilylmethyl vinyl carbonate and the like and mixtures thereof.

Another class of silicon-containing monomers includes polyurethane-polysiloxane macromonomers (also sometimes referred to as prepolymers), which may have hard-soft-hard blocks like traditional urethane elastomers. They may be end-capped with a hydrophilic monomer such as 2-hydroxyethyl methacrylate (HEMA). Examples of such silicone urethanes are disclosed in a variety or publications, including Lai, Yu-Chin, "The Role of Bulky Polysiloxanylalkyl Methacryates in Polyurethane-Polysiloxane Hydrogels, "Journal of Applied Polymer Science, Vol. 60, 1193-1199 (1996). PCT Published Application No. WO 96/31792 discloses examples of such monomers, which disclosure is hereby incorporated by reference in its entirety. Further examples of silicone urethane monomers are represented by Formulae II and III:

E(*D*A*D*G)ₐ *D*A*D*E'; (II)

or

E(*D*G*D*A)ₐ *D*A*D*E'; (III)

or
wherein:
D independently denotes an alkyl diradical, an alkyl cycloalkyl diradical, a cycloalkyl diradical, an aryl diradical or an alkylaryl diradical having 6 to about 30 carbon atoms;
G independently denotes an alkyl diradical, a cycloalkyl diradical, an alkyl cycloalkyl diradical, an aryl diradical or an alkylaryl diradical having 1 to about 40 carbon atoms and which may contain ether, thio or amine linkages in the main chain;
* denotes a urethane or ureido linkage;
a is at least 1;
A independently denotes a divalent polymeric radical of Formula IV: 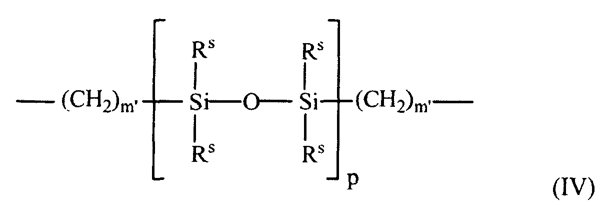 wherein each R^{s} independently denotes an alkyl or fluoro-substituted alkyl group having 1 to about 10 carbon atoms which may contain ether linkages between the carbon atoms; m' is at least 1; and p is a number that provides a moiety weight of about 400 to about 10,000;
each of E and E' independently denotes a polymerizable unsaturated organic radical represented by Formula V: 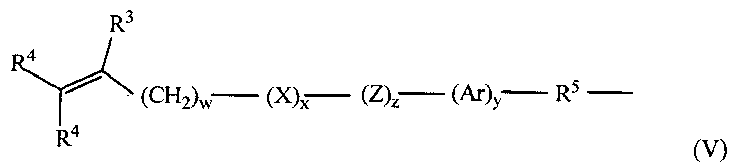
   wherein: R³ is hydrogen or methyl;
   R⁴ is hydrogen, an alkyl radical having 1 to 6 carbon atoms, or a -CO-Y-R⁶ radical wherein Y is -O-, -S- or NH-;
   R⁵ is a divalent alkylene radical having 1 to about 10 carbon atoms;
   R⁶ is a alkyl radical having 1 to about 12 carbon atoms;
   X denotes -CO- or -OCO-;
   Z denotes -O- or -NH-;
   Ar denotes an aromatic radical having about 6 to about 30 carbon atoms;
   w is 0 to 6; x is 0 or 1; y is 0 or 1; and z is 0 or 1.

A preferred silicone-containing urethane monomer is represented by Formula VI: 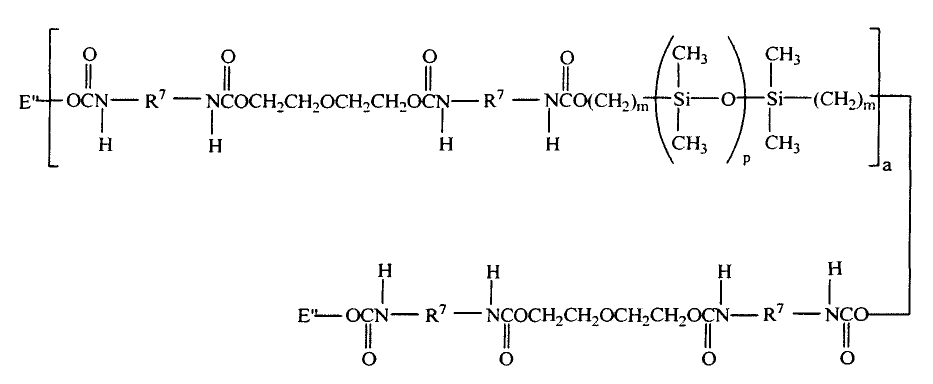 wherein m is at least 1 and is preferably 3 or 4, a is at least 1 and preferably is 1, p is a number which provides a moiety weight of about 400 to about 10,000 and is preferably at least about 30, R⁷ is a diradical of a diisocyanate after removal of the isocyanate group, such as the diradical of isophorone diisocyanate, and each E" is a group represented by:

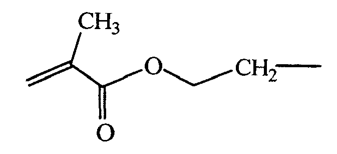

In another embodiment of the present invention, a silicone hydrogel material comprises (in bulk, that is, in the monomer mixture that is copolymerized) about 5 to about 50 percent, and preferably about 10 to about 25, by weight of one or more silicone macromonomers, about 5 to about 75 percent, and preferably about 30 to about 60 percent, by weight of one or more polysiloxanylalkyl (meth)acrylic monomers, and about 10 to about 50 percent, and preferably about 20 to about 40 percent, by weight of a hydrophilic monomer. In general, the silicone macromonomer is a poly(organosiloxane) capped with an unsaturated group at two or more ends of the molecule. In addition to the end groups in the above structural formulas, U.S. Patent No. 4,153,641 discloses additional unsaturated groups, including acryloxy or methacryloxy. Fumarate-containing materials such as those disclosed in U.S. Patent Nos. 5,310,779; 5,449,729 and 5,512,205 are also useful substrates in accordance with the invention. Preferably, the silane macromonomer is a silicon-containing vinyl carbonate or vinyl carbamate or a polyurethane-polysiloxane having one or more hard-soft-hard blocks and end-capped with a hydrophilic monomer.

The above silicone materials are merely exemplary, and other materials for use as substrates that can benefit by being packaged in the packaging solution according to the present invention and have been disclosed in various publications and are being continuously developed for use in contact lenses and other medical devices can also be used. For example, an ophthalmic lens for use herein can be a cationic lens such as a cationic contact lens or fluorinated silicone-containing monomers. Such monomers have been used in the formation of fluorosilicone as disclosed in, for example, U.S. Patent Nos. 4,954,587; 5,010,141 and 5,079,319. The use of silicone-containing monomers having certain fluorinated side groups, i.e., -(CF2)-H, have been found to improve compatibility between the hydrophilic and silicone-containing monomeric units. See, e.g., U.S. Patent Nos. 5,321,108 and 5,387,662.

Ophthalmic devices such as contact lenses for application of the present invention can be manufactured employing various conventional techniques, to yield a shaped article having the desired posterior and anterior lens surfaces. In one embodiment, an ophthalmic device can be prepared by polymerizing the monomeric mixtures to form a product that can be subsequently formed into the appropriate shape by, for example, lathing, injection molding, compression molding, cutting and the like. For example, in producing contact lenses, the initial monomeric mixture may be polymerized in tubes to provide rod-shaped articles, which are then cut into buttons. The buttons may then be lathed into contact lenses.

Alternately, the ophthalmic devices may be cast directly in molds, e.g., polypropylene molds, from the monomeric mixtures, e.g., by spincasting and static casting methods. Spincasting methods are disclosed in U.S. Patent Nos. 3,408,429 and 3,660,545, and static casting methods are disclosed in U.S. Patent Nos. 4,113,224, 4,197,266, and 5,271,875. Spincasting methods involve charging the monomer mixture to a mold, and spinning the mold in a controlled manner while exposing the monomer mixture to a radiation source such as UV light. Static casting methods involve charging the monomeric mixture between two mold sections, one mold section shaped to form the anterior lens surface and the other mold section shaped to form the posterior lens surface, and curing the monomeric mixture while retained in the mold assembly to form a lens, for example, by free radical polymerization of the monomeric mixture. Examples of free radical reaction techniques to cure the lens material include thermal radiation, infrared radiation, electron beam radiation, gamma radiation, ultraviolet (UV) radiation, and the like; or combinations of such techniques may be used. U.S. Patent No. 5,271,875 describes a static cast molding method that permits molding of a finished lens in a mold cavity defined by a posterior mold and an anterior mold. As an additional method, U.S. Patent No. 4,555,732 discloses a process where an excess of a monomeric mixture is cured by spincasting in a mold to form a shaped article having an anterior lens surface and a relatively large thickness, and the posterior surface of the cured spincast article is subsequently lathed to provide a contact lens having the desired thickness and posterior lens surface.

Polymerization may be facilitated by exposing the mixture to heat and/or radiation, such as ultraviolet light, visible light, or high energy radiation. A polymerization initiator may be included in the mixture to facilitate the polymerization step. Representative examples of free radical thermal polymerization initiators include organic peroxides such as acetal peroxide, lauroyl peroxide, decanoyl peroxide, stearoyl peroxide, benzoyl peroxide, tertiarylbutyl peroxypivalate, peroxydicarbonate, and the like. Representative UV initiators are those known in the art and include benzoin methyl ether, benzoin ethyl ether, Darocure 1173, 1164, 2273, 1116, 2959, 3331 (EM Industries) and Igracure 651 and 184 (Ciba-Geigy), and the like. Generally, the initiator will be employed in the monomeric mixture at a concentration of about 0.01 to 1 percent by weight of the total mixture.

After producing a lens having the desired final shape, it is desirable to remove residual solvent from the lens before edge-finishing operations. This is because, typically, an organic diluent is included in the initial monomeric mixture in order to minimize phase separation of polymerized products produced by polymerization of the monomeric mixture and to lower the glass transition temperature of the reacting polymeric mixture, which allows for a more efficient curing process and ultimately results in a more uniformly polymerized product. Sufficient uniformity of the initial monomeric mixture and the polymerized product are of particular concern for silicone hydrogels, primarily due to the inclusion of silicone-containing monomers which may tend to separate from the hydrophilic comonomer. Suitable organic diluents include, for example, monohydric alcohols such as C₆-C₁₀ straight-chained aliphatic monohydric alcohols, e.g., n-hexanol and n-nonanol; diols such as ethylene glycol; polyols such as glycerin; ethers such as diethylene glycol monoethyl ether; ketones such as methyl ethyl ketone; esters such as methyl enanthate; and hydrocarbons such as toluene. Preferably, the organic diluent is sufficiently volatile to facilitate its removal from a cured article by evaporation at or near ambient pressure. Generally, the diluent is included at about 5 to about 60 percent by weight of the monomeric mixture, with about 10 to about 50 percent by weight being especially preferred.

The cured lens can then be subjected to solvent removal, which can be accomplished by evaporation at or near ambient pressure or under vacuum. An elevated temperature can be employed to shorten the time necessary to evaporate the diluent. The time, temperature and pressure conditions for the solvent removal step will vary depending on such factors as the volatility of the diluent and the specific monomeric components, as can be readily determined by one skilled in the art. According to a preferred embodiment, the temperature employed in the removal step is preferably at least about 50°C, for example, about 60°C to about 80°C. A series of heating cycles in a linear oven under inert gas or vacuum may be used to optimize the efficiency of the solvent removal. The cured article after the diluent removal step should contain no more than twenty percent by weight of diluent, preferably no more than about 5 percent by weight or less.

Following removal of the organic diluent, the lens can then be subjected to mold release and optional machining operations. The machining step includes, for example, buffing or polishing a lens edge and/or surface. Generally, such machining processes may be performed before or after the article is released from a mold part. Preferably, the lens is dry released from the mold by employing vacuum tweezers to lift the lens from the mold, after which the lens is transferred by means of mechanical tweezers to a second set of vacuum tweezers and placed against a rotating surface to smooth the surface or edges. The lens may then be turned over in order to machine the other side of the lens

Next, the ophthalmic device such as a lens will be immersed in an aqueous packaging solution and stored in a packaging system according to the present invention. Generally, a packaging system for the storage of an ophthalmic device according to the present invention includes at least a sealed container containing one or more unused ophthalmic devices immersed in an aqueous packaging solution. Preferably, the sealed container is a hermetically sealed blister-pack, in which a concave well containing an ophthalmic device such as a contact lens is covered by a metal or plastic sheet adapted for peeling in order to open the blister-pack. The sealed container may be any suitable generally inert packaging material providing a reasonable degree of protection to the lens, preferably a plastic material such as polyalkylene, PVC, polyamide, and the like.

The aqueous packaging solution will contain at least a hydrophilic polymer having one or more non-ethylenically-unsaturated carboxylic acid terminated groups. The devices may either be unhydrated or prehydrated in water or an aqueous solution. The hydrophilic polymers can have a weight average molecular weight ranging from about 1,000 to about 1,000,000 and preferably from about 5,000 to about 100,000.

One class of a hydrophilic polymer having one or more non-ethylenically-unsaturated carboxylic acid terminated groups can be of the general formula: 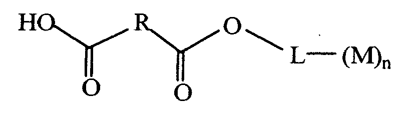 wherein R is a residue of a non-ethylenically-unsaturated carboxylic acid group-forming monomer, e.g., a saturated anhydride as discussed below, L is a linking group such as a direct bond or a divalent linkage group that includes a hydrocarbon group or a heterohydrocarbon group containing one or more atoms selected from the group consisting of O, N, S, and combinations thereof; M is an oligomer or polymer having a plurality of hydrophilic groups and n is an integer from 5 to about 10,000 and preferably about 50 to about 1,000. Representative examples of suitable L groups include an unsaturated or saturated, unsubstituted or substituted C₁-C₁₀ branched hydrocarbon group optionally containing one or more heteroatoms, an unsaturated or saturated, unsubstituted or substituted C₃-C₁₀ linear hydrocarbon group optionally containing one or more heteroatoms, an unsaturated or saturated, unsubstituted or substituted C₃-C₂₅ cyclic hydrocarbon group optionally containing one or more heteroatoms and the like.

Another class of a hydrophilic polymer having one or more non-ethylenically-unsaturated carboxylic acid terminated groups can be of the general formula: 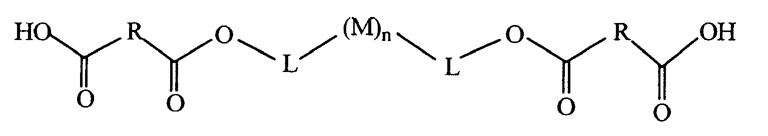 wherein R, L, M and n independently have the aforestated meanings.

The hydrophilic polymer having one or more non-ethylenically-unsaturated carboxylic acid terminated groups can be obtained by reacting a hydroxyl-terminated oligomer or polymer having a plurality of hydrophilic groups with a non-ethylenically-unsaturated carboxylic acid group-forming monomer such as a saturated cyclic anhydride under conditions effective to produce the hydrophilic polymer having one or more non-ethylenically-unsaturated carboxylic acid terminated groups. In one embodiment, the hydroxyl-terminated oligomer or polymer is a monohydroxyl-terminated oligomer or polymer. In another embodiment, the hydroxyl-terminated oligomer or polymer is a diterminal hydroxyl-terminated oligomer or polymer, i.e., the oligomer or polymer is terminated on each end with a hydroxyl-containing terminal group.

The oligomeric or polymeric chain comprises units derived from N-vinylpyrrolidone. In another embodiment, the oligomeric or polymeric chain comprises units are derived from mono- or polyhydric alcohols, e.g., glyceryl methacrylate, glyceryl acrylate, HEMA, erythritol (meth)acrylate, xylitol (meth)acrylate, sorbitol (meth)acrylate, and the like, acrylamides, e.g., dimethyl methacrylamide, DMA and the like, copolymers thereof and derivatives thereof. The oligomeric or polymeric chain can also comprises units derived from one of the foregoing monomers of an alkylene oxide (such as ethylene oxide).

The hydroxyl-terminated hydrophilic polymer can be synthesized by, for example, (a) mixing one or more oligomeric or polymeric chain-forming monomers or polymers with a hydroxyl-containing monomer; (b) adding a polymerization initiator; and (c) subjecting the monomer/initiator mixture to thermal energy or a source of ultraviolet or other light and curing the mixture. The mixture preferably contains at least one hydroxyl-containing monomer so that the polymer formed is hydroxyl-terminated. Useful hydroxyl-containing monomers include, by way of example, 2-isopropoxyethanol, allyl alcohol and the like.

Polymerization initiators include free-radical-generating polymerization initiators and ultraviolet (UV) free-radical initiators. Representative free radical thermal polymerization initiators are usually peroxides or azo initiators such as, for example, acetal peroxide, lauroyl peroxide, decanoyl peroxide, stearoyl peroxide, benzoyl peroxide, tertiarylbutyl peroxypivalate, peroxydicarbonate, 2,2'-azo-bis(2-methylpropionitrile), benzoin methyl ether and the like and mixtures thereof. Representative UV initiators are those known in the field such as, for example, benzoin methyl ether, benzoin ethyl ether, Darocure 1173, 1164, 2273, 1116, 2959, 3331 (EM Industries) and Igracure 651 and 184 (Ciba-Geigy), and the like and mixtures thereof. Other polymerization initiators which may be used are disclosed in, for example, "Polymer Handbook", 4th edition, Ed. J. Brandrup, E. H. Immergut, E. A. Grulke, A. Abe and D. R. Bloch, Pub. Wiley-Interscience, New York, 1998. The curing process will of course depend upon the initiator used and the physical characteristics of the comonomer mixture such as viscosity. In any event, the level of initiator employed may vary within the range of about 0.01 to about 2 weight percent of the mixture of monomers.

Polymerization of the mixture to form the hydroxyl-terminated hydrophilic polymer can be carried out in the presence of a solvent. Suitable solvents are in principle all solvents which dissolve the reactants such as, for example, water, alcohols such as lower alkanols, e.g., methanol, methanol and the like; carboxamides such as dimethylformamide and the like; dipolar aprotic solvents such as dimethyl sulfoxide and the like; ketones such as acetone, methyl ethyl ketone, cyclohexanone, and the like; aliphatic or aromatic hydrocarbons such as toluene, xylene, n-hexane and the like; ethers such as tetrahydrofuran, dimethoxyethane, dioxane and the like; halogenated hydrocarbons such as trichloroethane and the like, and also mixtures of suitable solvents, for example mixtures of water and an alcohol, e.g., a water/methanol or water/ethanol mixture, and the like.

Suitable saturated anhydrides are saturated, cyclic anhydrides. Preferably, the anhydride ring incorporates from two to four methylene or substituted methylene groups. Representative examples of such saturated, cyclic anhydrides include succinic anhydride, glutaric anhydride, adipic anhydride, methylsuccinic anhydride, 2-phenylglutaric anhydride, 3-methylglutaric anhydride, 3-methyladipic anhydride, and the like, and mixtures thereof.

The hydrophilic polymer having one or more non-ethylenically-unsaturated carboxylic acid terminated groups can be obtained by, for example, (a) reacting a hydroxyl-terminated oligomer or polymer having a plurality of hydrophilic groups with a non-ethylenically-unsaturated carboxylic acid group-forming monomer optionally in the presence of a polymerization initiator and (b) subjecting the monomer/initiator mixture to thermal energy or a source of ultraviolet or other light and curing the mixture. Polymerization initiators which may be used include the free-radical-generating polymerization initiators and UV free-radical initiators discussed above. The curing process will of course depend upon the initiator used and the physical characteristics of the comonomer mixture such as viscosity. In any event, the level of initiator employed may vary within the range of about 0.01 to about 2 weight percent of the mixture of monomers.

The relative amounts of hydroxyl-terminated hydrophilic polymer and the saturated, cyclic anhydride can vary over a fairly broad range. The amounts can be chosen to provide a polymer having the molecular weights discussed above. Generally, the anhydride hydroxyl-terminated hydrophilic polymer ratio is about 2:1.

Polymerization of the mixture to form the hydrophilic polymer can be carried out in the presence of a solvent. Suitable solvents are in principle all solvents which can dissolve the reactants such as, for example, water, alcohols such as lower alkanols, e.g., methanol, methanol and the like; carboxamides such as dimethylformamide and the like; dipolar aprotic solvents such as dimethyl sulfoxide and the like; ketones such as acetone, methyl ethyl ketone, cyclohexanone and the like; aliphatic or aromatic hydrocarbons such as toluene, xylene, n-hexane and the like; ethers such as tetrahydrofuran, dimethoxyethane, dioxane and the like; halogenated hydrocarbons such as trichloroethane and the like, and mixtures thereof, e.g., mixtures of water and an alcohol, e.g., a water/methanol or water/ethanol mixture, and the like.

The amount of the hydrophilic polymer employed in a packaging solution for storing an ophthalmic device in a packaging system of the present invention is an amount effective to improve the surface properties of the ophthalmic device. Generally, the concentration of a hydrophilic polymer present in the packaging solution of the invention will range from about 0.01 to about 10 % w/w.

The packaging solutions according to the present invention are physiologically compatible. Specifically, the solution must be "ophthalmically safe" for use with a lens such as a contact lens, meaning that a contact lens treated with the solution is generally suitable and safe for direct placement on the eye without rinsing, that is, the solution is safe and comfortable for daily contact with the eye via a contact lens that has been wetted with the solution. An ophthalmically safe solution has a tonicity and pH that is compatible with the eye and includes materials, and amounts thereof, that are non-cytotoxic according to ISO standards and U.S. Food & Drug Administration (FDA) regulations.

The packaging solution should also be sterile in that the absence of microbial contaminants in the product prior to release must be statistically demonstrated to the degree necessary for such products. The liquid media useful in the present invention are selected to have no substantial detrimental effect on the lens being treated or cared for and to allow or even facilitate the present lens treatment or treatments. The liquid media are preferably aqueous-based. A particularly useful aqueous liquid medium is that derived from saline, for example, a conventional saline solution or a conventional buffered saline solution.

The pH of the present solutions should be maintained within the range of about 6 to about 9, and preferably about 6.5 to about 7.8. Suitable buffers may be added, such as boric acid, sodium borate, potassium citrate, citric acid, sodium bicarbonate, TRIS and various mixed phosphate buffers (including combinations of Na2 HPO4, NaH2 PO4 and KH2 PO4) and mixtures thereof. Generally, buffers will be used in amounts ranging from about 0.05 to about 2.5 percent by weight, and preferably from about 0.1 to about 1.5 percent by weight of the solution. The packaging solutions of this invention preferably contain a borate buffer, containing one or more of boric acid, sodium borate, potassium tetraborate, potassium metaborate or mixtures of the same.

Typically, the solutions of the present invention are also adjusted with tonicity agents, to approximate the osmotic pressure of normal lacrimal fluids which is equivalent to a 0.9 percent solution of sodium chloride or 2.5 percent of glycerol solution. The solutions are made substantially isotonic with physiological saline used alone or in combination, otherwise if simply blended with sterile water and made hypotonic or made hypertonic the lenses will lose their desirable optical parameters. Correspondingly, excess saline may result in the formation of a hypertonic solution which will cause stinging and eye irritation.

Examples of suitable tonicity adjusting agents include, but are not limited to, sodium and potassium chloride, dextrose, glycerin, calcium and magnesium chloride and the like and mixtures thereof. These agents are typically used individually in amounts ranging from about 0.01 to about 2.5% w/v and preferably from about 0.2 to about 1.5% w/v. Preferably, the tonicity agent will be employed in an amount to provide a final osmotic value of at least about 200 mOsm/kg, preferably from about 200 to about 400 mOsm/kg, more preferably from about 250 to about 350 mOsm/kg, and most preferably from about 280 to about 320 mOsm/kg.

If desired, one or more additional components can be included in the packaging solution. Such additional component or components are chosen to impart or provide at least one beneficial or desired property to the packaging solution. Such additional components may be selected from components which are conventionally used in one or more ophthalmic device care compositions. Examples of such additional components include cleaning agents, wetting agents, nutrient agents, sequestering agents, viscosity builders, contact lens conditioning agents, antioxidants, and the like and mixtures thereof. These additional components may each be included in the packaging solutions in an amount effective to impart or provide the beneficial or desired property to the packaging solutions. For example, such additional components may be included in the packaging solutions in amounts similar to the amounts of such components used in other, e.g., conventional, contact lens care products.

Useful sequestering agents include, but are not limited to, disodium ethylene diamine tetraacetate, alkali metal hexametaphosphate, citric acid, sodium citrate and the like and mixtures thereof.

Useful viscosity builders include, but are not limited to, hydroxyethyl cellulose, hydroxymethyl cellulose, polyvinyl pyrrolidone, polyvinyl alcohol and the like and mixtures thereof.

Useful antioxidants include, but are not limited to, sodium metabisulfite, sodium thiosulfate, N-acetylcysteine, butylated hydroxyanisole, butylated hydroxytoluene and the like and mixtures thereof.

The method of packaging and storing an ophthalmic device such as a contact lens according to the present invention includes at least packaging an ophthalmic device immersed in the aqueous packaging solution described above. The method may include immersing the ophthalmic device in an aqueous packaging solution prior to delivery to the customer/wearer, directly following manufacture of the contact lens. Alternately, the packaging and storing in the solution of the present invention may occur at an intermediate point before delivery to the ultimate customer (wearer) but following manufacture and transportation of the lens in a dry state, wherein the dry lens is hydrated by immersing the lens in the contact-lens packaging solution. Consequently, a package for delivery to a customer may include a sealed container containing one or more unused contact lenses immersed in an aqueous packaging solution according to the present invention.

In one embodiment, the steps leading to the present ophthalmic device packaging system includes (1) molding an ophthalmic device in a mold comprising at least a first and second mold portion, (2) hydrating and cleaning the device in a container comprising at least one of the mold portions, (3) introducing the packaging solution with the hydrophilic polymer having one or more non-ethylenically-unsaturated carboxylic acid terminated groups into the container with the device supported therein, and (4) sealing the container. Preferably, the method also includes the step of sterilizing the contents of the container. Sterilization may take place prior to, or most conveniently after, sealing of the container and may be effected by any suitable method known in the art, e.g., by autoclaving of the sealed container at temperatures of about 120°C or higher.

The following examples are provided to enable one skilled in the art to practice the invention and are merely illustrative of the invention. The examples should not be read as limiting the scope of the invention as defined in the claims.

In the examples, the following abbreviations are used.
I4D5S4H: A prepolymer derived from 10 moles of isophorone diisocyanate, 4 moles of diethyleneglycol, 5 moles of hydroxybutyl-terminated polydimethylsiloxane of Mn 4000 and end-capped with 2-hydroxyethyl methacrylate
TRIS: tris(trimethylsiloxy)silylpropyl methacrylate
NVP: N-vinyl-2-pyrrolidone
PVP: poly(vinylpyrrolidone)
DMA: N,N-dimethyl acrylamide
HEMA: 2-hydroxyethyl methacrylate
HEMAVC: methacryloxyethyl vinyl carbonate
D1173: 2-hydroxy-2-methyl-1-phenylpropan-1-one (available as Darocur 1173 initiator)
IMVT: 1,4-bis(4-(2-methacryloxyethyl)phenylamino)anthraquinone
PP: polypropylene
DI: de-ionized water
IPA: isopropyl alcohol
AIBN: azo bis-isobutylnitrile (commercially available as VazoTM 64)
THF: tetrahydrofuran

### EXAMPLE 1

### Preparation of hydroxyl functionalized poly (vinyl pyrrolidone).

To a 2-liter three-neck flask equipped with a condenser and nitrogen inlet tube was added 900 ml of 2-isopropoxyethanol (about 813.6 g, 7.812 mol, Aldrich Chemical Company), 30 mol of freshly distilled NVP (about 31.35 g, 0.282 mol) and AIBN (0.317 g; 1.930 mmol). The contents were bubbled vigorously with nitrogen for 1 hour. Then, while under nitrogen blanket, the contents were heated at 80°C for two days. The solution was ultra filtrated using a 1000 NMWL RC film. Next, the solvent was removed using a rotavapor at 50 to 60 rpms. THF (100 ml) was added to dissolve the product and then tin 2000 mL ether was poured to precipitate the product. The product was dried in a vacuum oven to give 30.85 g of product. The hydroxyl functionalized PVP had a number average molecular weight (Mₙ) of 1357 as determined by titration.

### EXAMPLE 2

### Preparation of acid-terminated PVP.

To a thoroughly dried 500-mL round bottom flask equipped with nitrogen inlet tube and drying tube, is charged 200 mL of anhydrous THF. Next, succinic anhydride (2.00 g, 0.02 mole) and the hydroxyl functionalized poly(vinyl pyrrolidone) (13.57 g, 0.010 mole) of Example 1 are added to the flask. The contents are heated under reflux for 48 hours with stirring. The solution is concentrated to 100 mL and is poured into 2000 mL of ether to precipitate the product.

### EXAMPLE 3

### Preparation of poly (vinyl pyrrolidone-co-allyl alcohol).

To a 1000 ml three-neck flask equipped with a condenser and nitrogen inlet tube was added 250 mL of distilled water, 45.51 g (409.5 mmole) of freshly distilled NVP, 1,1725 g (20.19 mmole) of allyl alcohol and AIBN (0.47 g; 2.862 mmol). The contents were bubbled vigorously with nitrogen for 1 hour. While under nitrogen blanket and with stirring, the contents were heated up to 70°C for two days. The solution became viscous even after one hour of heating. After two days, the product was recovered by freeze drying. The product had a Mn of 1,020,00, a Mw of 1,355,000 and a polydispersity of 1.327, as determined by Size Exclusion Chromatography. It was found that there was 1 allyl alcohol per 100 vinylpyrrolidone units.

### EXAMPLE 4

### Preparation of acid-terminated PVP.

To a thoroughly dried 2-L round bottom flask equipped with nitrogen inlet tube and drying tube is charged with 30 g of product from Example 3 and 300 ml of anhydrous THF 200. The solution is refluxed until full solution. Next, succinic anhydride (0.9 g, 0.009 mole) is added to the flask. The contents are heated under reflux for 48 hours with stirring. The solution is then concentrated to 100 mL and is poured into 2 liter ether to precipitate the product.

### EXAMPLE 5

### Preparation of a polyurethane-siloxane hydrogel lens.

A monomer mixture was made by mixing the following components listed in Table 1, at amounts per weight.

**TABLE 1**

| Ingredient | Amount |
|---|---|
| I4D5S4H | 53 |
| TRIS | 15 |
| DMA | 9 |
| NVP | 24 |
| HEMA | 5 |
| HEMAVC | 1 |
| Hexanol | 10 |
| Darocur-1173 | 0.5 |
| IMVT | 150 ppm |

Lenses were cast using polypropylene molds, both in an open air bench top and in a dry box filled with nitrogen, and then cured in an oven at under the following thermal conditions: held at room temperature for 12 minutes, then ramped up to 100°C in 54 minutes, and further held at 100°C for 2 hours. After casting, the lenses were released from the molds and extracted in IPA for 2 hours. The lenses were rinsed with distilled water and then placed in borate buffered saline. Some of these lenses were autoclaved for one cycle.

### EXAMPLE 6

An aqueous packaging solution containing 3% by weight of the acid-terminated PVP of Example 2 dissolved in a borate buffered saline at a pH of 7.2 is placed in a polypropylene blister package. Next, the lenses of Example 5 are immersed in the aqueous packaging solution in the polypropylene blister package. The package is sealed with foil lidstock and then autoclaved for 1 cycle.

### EXAMPLE 7

An aqueous packaging solution containing 3% by weight of the acid-terminated PVP of Example 4 dissolved in a borate buffered saline at a pH of 7.2 is placed in a polypropylene blister package. Next, the lenses of Example 5 are immersed in the aqueous packaging solution in the polypropylene blister package. The package is sealed with foil lidstock and then autoclaved for 1 cycle.

### EXAMPLE 8

An aqueous packaging solution containing 1% by weight of the acid-terminated PVP of Example 1 dissolved in a borate buffered saline at a pH of 7.2 is placed in a polypropylene blister package. Next, a balafilcon A contact lens (a commercially available group III extended wear contact lenses from Bausch & Lomb Incorporated of Rochester, N.Y., sold under the trade name Purevision^{®}, made of a silicone hydrogel material and having an anionic charge and approximately 38% water) is immersed in the aqueous packaging solution in the polypropylene blister package. The package is sealed with foil lidstock and then autoclaved for 1 cycle.

### EXAMPLE 9

An aqueous packaging solution containing 1% by weight of the acid-terminated PVP of Example 4 dissolved in a borate buffered saline at a pH of 7.2 is placed in a polypropylene blister package. Next, a balafilcon A contact lens (a commercially available group III extended wear contact lenses from Bausch & Lomb Incorporated of Rochester, N.Y., sold under the trade name Purevision^{®}, made of a silicone hydrogel material and having an anionic charge and approximately 38% water) is immersed in the aqueous packaging solution in the polypropylene blister package. The package is sealed with foil lidstock and then autoclaved for 1 cycle.

### EXAMPLE 10

An aqueous packaging solution containing 2% by weight of the acid-terminated PVP of Example 1 dissolved in a borate buffered saline at a pH of 7.2 is placed in a polypropylene blister package. Next, a balafilcon A contact lens (a commercially available group III extended wear contact lenses from Bausch & Lomb Incorporated of Rochester, N.Y., sold under the trade name Purevision^{®}, made of a silicone hydrogel material and having an anionic charge and approximately 38% water) is immersed in the aqueous packaging solution in the polypropylene blister package. The package is sealed with foil lidstock and then autoclaved for 1 cycle.

### EXAMPLE 11

An aqueous packaging solution containing 2% by weight of the acid-terminated PVP of Example 4 dissolved in a borate buffered saline at a pH of 7.2 is placed in a polypropylene blister package. Next, a balafilcon A contact lens (a commercially available group III extended wear contact lenses from Bausch & Lomb Incorporated of Rochester, N.Y., sold under the trade name Purevision®, made of a silicone hydrogel material and having an anionic charge and approximately 38% water) is immersed in the aqueous packaging solution in the polypropylene blister package. The package is sealed with foil lidstock and then autoclaved for 1 cycle.

It will be understood that various modifications may be made to the embodiments disclosed herein. Therefore the above description should not be construed as limiting, but merely as exemplifications of preferred embodiments. For example, the functions described above and implemented as the best mode for operating the present invention are for illustration purposes only.

## Claims

1. A method of preparing a package comprising a storable, sterile ophthalmic device, the method comprising:
(a) immersing an ophthalmic device in an aqueous packaging solution comprising one or more hydrophilic polymers having one or more non-ethylenically-unsaturated carboxylic acid terminated groups, wherein the hydrophilic polymer comprises one or more units derived from N-vinylpyrrolidone, alkylene oxides, glyceryl methacrylate, glyceryl acrylate, dimethyl methacrylamide, dimethyl acrylamide, 2-hydroxyethyl methacrylate, 2-hydroxyethyl acrylate, erythritol methacrylate, erythritol acrylate, xylitol methacrylate, xylitol acrylate, sorbitol methacrylate, sorbitol acrylate, or mixtures thereof, and wherein the solution has an osmolality of at least 200 mOsm/kg and a pH in the range of 6 to 9;
(b) packaging the solution and the device in a manner preventing contamination of the device by microorganisms; and
(c) sterilizing the packaged solution and device.

2. The method of claim 1, wherein the ophthalmic device is a contact lens.

3. The method of claim 1, wherein the ophthalmic device is a silicone hydrogel contact lens.

4. The method of claim 1, wherein the hydrophilic polymer comprises one or more units derived from N-vinylpyrrolidone.

5. The method of claim 1, wherein the hydrophilic polymer is a reaction product of a hydroxyl-terminated oligomer or polymer having a plurality of hydrophilic groups with a saturated, cyclic anhydride.

6. The method of claim 5, wherein the saturated, cyclic anhydride is selected from the group consisting of succinic anhydride, glutaric anhydride, adipic anhydride and mixtures thereof.

7. The method of claim 1, wherein the hydrophilic polymer has one non-ethylenically-unsaturated carboxylic acid terminated group.

8. The method of claim 1, wherein the hydrophilic polymer is terminated on each end with a non-ethylenically-unsaturated carboxylic acid terminal group.

9. The method of claim 1, wherein the concentration of the hydrophilic polymer in the aqueous packaging solution is 0.01 to 10 % w/w.

10. The method of claim 1, wherein the solution does not contain an effective disinfecting amount of a disinfecting agent.

11. A packaging system for the storage of an ophthalmic device comprising a sealed container containing one or more unused ophthalmic devices immersed in an aqueous packaging solution comprising one or more hydrophilic polymers having one or more non-ethylenically-unsaturated carboxylic acid terminated groups, wherein the hydrophilic polymer comprises one or more units derived from N-vinylpyrrolidone, alkylene oxides, glyceryl methacrylate, glyceryl acrylate, dimethyl methacrylamide, dimethyl acrylamide, 2-hydroxyethyl methacrylate, 2-hydroxyethyl acrylate, erythritol methacrylate, erythritol acrylate, xylitol methacrylate, xylitol acrylate, sorbitol methacrylate, sorbitol acrylate, or mixtures thereof, and wherein the solution has an osmolality of at least 200 mOsm/kg, a pH of 6 to 9 and is heat sterilized.

12. The packaging system of claim 11, wherein the ophthalmic device is a contact lens.

13. The packaging system of claim 11, wherein the hydrophilic polymer comprises one or more units derived from N-vinylpyrrolidone.

14. The packaging system of claim 11, wherein the hydrophilic polymer is a reaction product of a hydroxyl-terminated oligomer or polymer having a plurality of hydrophilic groups with a saturated, cyclic anhydride.

15. The packaging system of claim 14, wherein the saturated, cyclic anhydride is selected from the group consisting of succinic anhydride, glutaric anhydride, adipic anhydride and mixtures thereof.

16. The packaging system of claim 11, wherein the hydrophilic polymer has one non-ethylenically-unsaturated carboxylic acid terminated group.

17. The packaging system of claim 11, wherein the hydrophilic polymer is terminated on each end with a non-ethylenically-unsaturated carboxylic acid terminal group.

18. The packaging system of claim 11, wherein the solution does not contain an effective disinfecting amount of a disinfecting agent.

19. The packaging system of claim 11, wherein the solution does not contain a germicide compound.

20. The packaging system of claim 11, wherein the package is heat sterilized subsequent to sealing of the package.

## Patentansprüche

1. Ein Verfahren zur Herstellung einer Verpackung welche eine lagerfähige, sterile ophthalmische Vorrichtung umfasst, das Verfahren umfassend:
(a) Einlegen einer ophthalmischen Vorrichtung in eine wässrige Verpackungslösung, umfassend ein oder mehrere hydrophile Polymere, welche eine oder mehrere nicht-ethylenisch-ungesättigte Carboxylsäure-terminierte Gruppen aufweisen, wobei die hydrophilen Polymere eine oder mehrere Einheiten abgeleitet von N-Vinylpyrrolidon, Alkylenoxid, Glycerylmethacrylat, Glycerylacrylat, Dimethylmethacrylamid, Dimethylacrylamid, 2-Hydroxyethylmethacrylat, 2-Hydroxyethylacrylat, Erythritolmethacrylat, Erythritolacrylat, Xylitolmethacrylat, Xylitolacrylat, Sorbitolmethacrylat, Sorbitolacrylat oder Mischungen daraus umfassen und wobei die Lösung eine Osmolalität von wenigstens 200 mOsm/kg und einen pH-Wert im Bereich von 6 bis 9 aufweist;
(b) Verpacken der Lösung und der Vorrichtung in einer Weise, welche eine Kontamination der Vorrichtung durch Mikroorganismen verhindert; und
(c) Sterilisieren der verpackten Lösung und Vorrichtung.

2. Das Verfahren gemäß Anspruch 1, wobei die ophthalmische Vorrichtung eine Kontaktlinse ist.

3. Das Verfahren gemäß Anspruch 1, wobei die ophthalmische Vorrichtung eine Silikonhydrogel-Kontaktlinse ist.

4. Das Verfahren gemäß Anspruch 1, wobei das hydrophile Polymer eine oder mehrere Einheiten, abgeleitet von N-Vinylpyrrolidon, umfasst.

5. Das Verfahren gemäß Anspruch 1, wobei das hydrophile Polymer ein Reaktionsprodukt eines Hydroxyl-terminierten Oligomers oder Polymers ist, das eine Mehrzahl an hydrophilen Gruppen mit einem gesättigten, cyclischen Anhydrid aufweist.

6. Das Verfahren gemäß Anspruch 5, wobei das gesättigte, cyclische Anhydrid ausgewählt ist aus der Gruppe bestehend aus Bernsteinsäureanhydrid, Glutarsäureanhydrid, Adipinsäureanhydrid und Mischungen davon.

7. Das Verfahren gemäß Anspruch 1, wobei das hydrophile Polymer eine nicht-ethylenisch-ungesättigte Carboxylsäure-terminierte Gruppe aufweist.

8. Das Verfahren gemäß Anspruch 1, wobei das hydrophile Polymer an jedem Ende mit einer nicht-ethylenisch-ungesättigten Carboxylsäure-terminierten Gruppe terminiert ist.

9. Das Verfahren gemäß Anspruch 1, wobei die Konzentration des hydrophilen Polymers in der wässrigen Verpackungslösung 0,01 bis 10 % w/w ist.

10. Das Verfahren gemäß Anspruch 1, wobei die Lösung keine wirksam desinfizierende Menge eines Desinfektionsmittels enthält.

11. Ein Verpackungssystem zur Lagerung einer ophthalmischen Vorrichtung umfassend einen versiegelten Behälter, der eine oder mehrere unbenutzte ophthalmische Vorrichtungen enthält, eingelegt in eine wässrige Verpackungslösung, umfassend ein oder mehrere hydrophile Polymere, die eine oder mehrere nicht-ethylenisch-ungesättigte Carboxylsäure-terminierte Gruppen aufweisen, wobei die hydrophilen Polymere eine oder mehrere Einheiten abgeleitet von N-Vinylpyrrolidon, Alkylenoxid, Glycerylmethacrylat, Glycerylacrylat, Dimethylmethacrylamid, Dimethylacrylamid, 2-Hydroxyethylmethacrylat, 2-Hydroxyethylacrylat, Erythritolmethacrylat, Erythritolacrylat, Xylitolmethacrylat, Xylitolacrylat, Sorbitolmethacrylat, Sorbitolacrylat oder Mischungen daraus umfassen und wobei die Lösung eine Osmolalität von wenigstens 200 mOsm/kg und einen pH-Wert im Bereich von 6 bis 9 aufweist und hitzesterilisiert ist.

12. Das Verpackungssystem gemäß Anspruch 11, wobei die ophthalmische Vorrichtung eine Kontaktlinse ist.

13. Das Verpackungssystem gemäß Anspruch 11, wobei das hydrophile Polymer eine oder mehrere Einheiten, abgeleitet von N-Vinylpyrrolidon, umfasst.

14. Das Verpackungssystem gemäß Anspruch 11, wobei das hydrophile Polymer ein Reaktionsprodukt eines Hydroxyl-terminierten Oligomers oder Polymers ist, das eine Mehrzahl an hydrophilen Gruppen mit einem gesättigten, cyclischen Anhydrid aufweist.

15. Das Verpackungssystem gemäß Anspruch 14, wobei das gesättigte, cyclische Anhydrid ausgewählt ist aus der Gruppe bestehend aus Bernsteinsäureanhydrid, Glutarsäureanhydrid, Adipinsäureanhydrid und Mischungen davon.

16. Das Verpackungssystem gemäß Anspruch 11, wobei das hydrophile Polymer eine nicht-ethylenisch-ungesättigte Carboxylsäure-terminierte Gruppe aufweist.

17. Das Verpackungssystem gemäß Anspruch 11, wobei das hydrophile Polymer an jedem Ende mit einer nicht-ethylenisch-ungesättigten Carboxylsäure-terminierten Gruppe terminiert ist.

18. Das Verpackungssystem gemäß Anspruch 11, wobei die Lösung keine wirksam desinfizierende Menge eines Desinfektionsmittels enthält.

19. Das Verpackungssystem gemäß Anspruch 11, wobei die Lösung kein Germizid enthält.

20. Das Verpackungssystem gemäß Anspruch 11, wobei die Verpackung sofort nach dem Versiegeln der Verpackung hitzesterilisiert wird.

## Revendications

1. Procédé de préparation d'un produit conditionné comprenant un dispositif ophtalmique stérile stockable, le procédé comprenant :
(a) l'immersion d'un dispositif ophtalmique dans une solution de conservation aqueuse comprenant un ou plusieurs polymères hydrophiles comportant un ou plusieurs groupes à terminaison acide carboxylique non éthyléniquement insaturés, où le polymère hydrophile comprend une ou plusieurs unités dérivées de la N-vinylpyrrolidone, des oxydes d'alkylène, du méthacrylate de glycéryle, de l'acrylate de glycéryle, du méthacrylamide de diméthyle, de l'acrylamide de diméthyle, du méthacrylate de 2-hydroxyéthyle, de l'acrylate de 2-hydroxyéthyle, du méthacrylate d'érythritol, de l'acrylate d'érythritol, du méthacrylate de xylitol, de l'acrylate de xylitol, du méthacrylate de sorbitol, de l'acrylate de sorbitol ou leurs mélanges, et où la solution présente une osmolalité d'au moins 200 mOsm/kg et un pH dans la plage de 6 à 9 ;
(b) l'emballage de la solution et du dispositif d'une manière permettant de prévenir la contamination du dispositif par les microorganismes ; et
(c) la stérilisation de la solution et du dispositif conditionnés.

2. Procédé selon la revendication 1, dans lequel le dispositif ophtalmique est une lentille de contact.

3. Procédé selon la revendication 1, dans lequel le dispositif ophtalmique est une lentille de contact en silicone hydrogel.

4. Procédé selon la revendication 1, dans lequel le polymère hydrophile comprend une ou plusieurs unités dérivées de la N-vinylpyrrolidone.

5. Procédé selon la revendication 1, dans lequel le polymère hydrophile est un produit réactionnel d'un oligomère ou d'un polymère à terminaison hydroxyle comportant une pluralité de groupes hydrophiles avec un anhydride cyclique saturé.

6. Procédé selon la revendication 5, dans lequel l'anhydride cyclique saturé est choisi dans le groupe constitué de l'anhydride succinique, de l'anhydride glutarique, de l'anhydride adipique et de leurs mélanges.

7. Procédé selon la revendication 1, dans lequel le polymère hydrophile comporte un groupe à terminaison acide carboxylique non éthyléniquement insaturé.

8. Procédé selon la revendication 1, dans lequel le polymère hydrophile est terminé à chaque extrémité par un groupe à terminaison acide carboxylique non éthyléniquement insaturé.

9. Procédé selon la revendication 1, dans lequel la concentration du polymère hydrophile dans la solution de conservation aqueuse est de 0,01 à 10 % p/p.

10. Procédé selon la revendication 1, dans lequel la solution ne contient pas une quantité à effet désinfectant d'un agent désinfectant.

11. Système de conditionnement pour le stockage d'un dispositif ophtalmique comprenant un récipient fermé hermétiquement contenant un ou plusieurs dispositifs ophtalmiques non utilisés immergés dans une solution de conservation aqueuse comprenant un ou plusieurs polymères hydrophiles comportant un ou plusieurs groupes à terminaison acide carboxylique non éthyléniquement insaturés, où le polymère hydrophile comprend une ou plusieurs unités dérivées de la N-vinylpyrrolidone, des oxydes d'alkylène, du méthacrylate de glycéryle, de l'acrylate de glycéryle, du méthacrylamide de diméthyle, de l'acrylamide de diméthyle, du méthacrylate de 2-hydroxyéthyle, de l'acrylate de 2-hydroxyéthyle, du méthacrylate d'érythritol, de l'acrylate d'érythritol, du méthacrylate de xylitol, de l'acrylate de xylitol, du méthacrylate de sorbitol, de l'acrylate de sorbitol ou leurs mélanges et où la solution présente une osmolalité d'au moins 200 mOsm/kg, un pH de 6 à 9 et est stérilisée par la chaleur.

12. Système de conditionnement selon la revendication 11, dans lequel le dispositif ophtalmique est une lentille de contact.

13. Système de conditionnement selon la revendication 11, dans lequel le polymère hydrophile comprend une ou plusieurs unités dérivées de la N-vinylpyrrolidone.

14. Système de conditionnement selon la revendication 11, dans lequel le polymère hydrophile est un produit réactionnel d'un oligomère ou d'un polymère à terminaison hydroxyle comportant une pluralité de groupes hydrophiles avec un anhydride cyclique saturé.

15. Système de conditionnement selon la revendication 14, dans lequel l'anhydride cyclique saturé est choisi dans le groupe constitué de l'anhydride succinique, de l'anhydride glutarique, de l'anhydride adipique et de leurs mélanges.

16. Système de conditionnement selon la revendication 11, dans lequel le polymère hydrophile comporte un groupe à terminaison acide carboxylique non éthyléniquement insaturé.

17. Système de conditionnement selon la revendication 11, dans lequel le polymère hydrophile est terminé à chaque extrémité par un groupe à terminaison acide carboxylique non éthyléniquement insaturé.

18. Système de conditionnement selon la revendication 11, dans lequel la solution ne contient pas une quantité à effet désinfectant d'un agent désinfectant.

19. Système de conditionnement selon la revendication 11, dans lequel la solution ne contient pas de composé germicide.

20. Système de conditionnement selon la revendication 11, dans lequel le produit conditionné est stérilisé par la chaleur après la fermeture hermétique de l'emballage.
